# Europäisches Patentamt

# European Patent Office

# Office européen des brevets

(19)

(11) Veröffentlichungsnummer: **0 106 373**
**B1**

(12)

# EUROPÄISCHE PATENTSCHRIFT

(45) Veröffentlichungstag der Patentschrift:
14.05.86

(51) Int. Cl.⁴: **C 07 D 213/16, C 07 D 295/02**

(21) Anmeldenummer: 83200922.9

(22) Anmeldetag: 22.06.83

(54) Verfahren zur Raffination von wasserhaltigen Stickstoffheterocyclen und -gemischen.

(30) Priorität: 29.09.82 DE 3236002

(43) Veröffentlichungstag der Anmeldung:
25.04.84 Patentblatt 84/17

(45) Bekanntmachung des Hinweises auf die Patenterteilung:
14.05.86 Patentblatt 86/20

(84) Benannte Vertragsstaaten:
BE CH DE FR GB LI NL

(56) Entgegenhaltungen:
DD - C - 63 492
DE - C - 850 006

Chemical Abstracts Band 72, Nr. 18, 4. Mai 1970,
Columbus, Ohio, USA N.I. NIKUSASHINA et al. "Salting
out in three-component systems. III", Seite 270, Spalte
1, Abstract Nr. 93830v
Chemical Abstracts Band 72, Nr. 18, 4. Mai 1970,
Columbus, Ohio, USA R.V. MERTSLIN et al. "Salting out
in three-component systems. II.", Seite 270, Spalte 1,
Abstract Nr. 93831w

(73) Patentinhaber: Rütgerswerke Aktiengesellschaft,
Mainzer Landstrasse 217, D-6000 Frankfurt
a.Main 1 (DE)

(72) Erfinder: Grigoleit, Georg, Dr., Moselweg 7,
D-4270 Dorsten-19 (DE)
Erfinder: Oberkobusch, Rudolf, Dr., Am Botanischen
Garten 3, D-4100 Duisburg-1 (DE)
Erfinder: Stadelhofer, Jürgen, Dr.,
Groppenbrucherstrasse 21, D-4600 Dortmund-15 (DE)
Erfinder: Matern, Kurt, Quadtstrasse 9,
D-4100 Duisburg-12 (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neuartiges Verfahren zur Raffination von wasserhaltigen Stickstoffheterocyclen und -gemischen.

Stickstoffheterocyclen wie z.B. Piperidin, Pyridin oder Chinolin und ihre Alkylderivate sind wichtige Ausgangsverbindungen für die organische und speziell pharmazeutische Chemie. Diese Verbindungen werden zum Teil auf steinkohlenteerstämmiger Basis gewonnen. Der mengenmässige Grossteil der Stickstoffheterocyclen wird jedoch aus Sauerstoff enthaltenden Verbindungen durch Umsetzung mit Ammoniak hergestellt. Beispielhaft hierfür sind die Synthesen von Pyridin, das durch Reaktion von Ammoniak, Acetaldehyd und Formaldehyd gewonnen wird, sowie von Piperidin das durch Umsetzung von Tetrahydrofurfurylalkohol und Ammoniak herstellbar ist. Alkylsubstituierte Pyridine, die insbesondere für die Nikotinsäure- und Isonikotinsäure-Herstellung eingesetzt werden, werden gleichfalls durch Reaktion von Acetaldehyd und Ammoniak hergestellt. Bei diesen Verfahren fällt stets Wasser als Koppelprodukt an. Die Rohbasen müssen daher raffiniert werden, wobei das Wasser in einem Reinigungsprozess abgetrennt wird. Üblicherweise wird die Abtrennung des Wassers als destillative Entwässerung mit Schleppmitteln wie Benzol, Toluol, Diisobutyläther unter anderem durchgeführt. Dies ist sowohl bei tiefsiedenden Basen problematisch, deren Siedepunkt nahe dem des Wassers liegt, als auch bei den Basen, die mit Wasser ein Azeotrop bilden. Das abgetrennte Wasser enthält weiterhin Stickstoffheterocyclen.

Eine Extraktion der Basen mit unpolaren Lösungsmitteln bringt auch nach mehrfachen Wiederholungen keine vollständige Abtrennung von Wasser.

GB-PS 580 048 beschreibt ein Verfahren zur Trennung von Stickstoffbasen durch die Zugabe von zusätzlichem Wasser und anschliessende Azeotropdestillation des Gemisches, wodurch es möglich wird, die einzelnen Basen wie Lutidine und Picoline voneinander zu trennen. Die aufgetrennten Basenfraktionen enthalten danach Wasser, dessen Abtrennung durch Zugabe von festem Alkalihydroxid erfolgt. Dieses Verfahren ist ausserordentlich umständlich, da mehrmals festes Alkalihydroxid zugegeben und die sich dann bildende Lauge jeweils abgezogen werden muss. Eine kontinuierliche Abtrennung ist nicht möglich.

Es sind ferner Verfahren bekannt, Stickstoffheterocyclen durch Aussalzen, d.h. durch Zugabe wasserlöslicher Salze zum Stickstoffbasen-Wasser-Gemisch abzutrennen. Bedingt jedoch durch die hohe Affinität dieser Stickstoffheterocyclen zu Wasser bleibt ein grosser Teil dieser Verbindungen im Wasser gelöst. So verbleiben nach Nikurashima, N.I.; Dudkin, A.S. (USSR). Fiz.-Khim. Issled. Svoistv Prostykh Kompleks. Soedin. RZE (Redkozemel. Elem.) Troinykh Sist. Razlichnym Vzaimodeishiem Komponentov 1968, 15-28 (referiert in C.A. <u>72</u> (1970) 93830 und 93831 w) beim Aussalzen von Pyridin-Wasser-Gemischen mit NaCl noch 5,66% und mit KCl noch 10% Pyridin im Wasser.

Es bestand daher die Aufgabe, ein Verfahren zur Raffination von Stickstoffheterocyclen aus wasserhaltigen Gemischen zu entwickeln, bei dem das Wasser durch möglichst wenig einfache Verfahrensschritte und gegebenenfalls auch kontinuierlich praktisch quantitativ von der organischen Phase abgetrennt werden kann, ohne dass ein nennenswerter Anteil der Basen im Wasser verbleibt. Diese Aufgabe wird gelöst durch ein Verfahren gemäss der Ansprüche 1 - 7.

Überraschenderweise wurde gefunden, dass eine praktisch quantitative Abscheidung von Basen möglich ist, wenn man eine Kombination von wasserlöslichem Salz und einem aliphatischen oder cycloaliphatischen Lösungsmittel anwendet.

Dadurch werden mit einem einfachen Verfahren die Stickstoffheterocyclen wasserfrei erhalten in nahezu quantitativer Ausbeute. Andererseits ist die wässrige Phase basenfrei und kann ohne Umweltbelastung verworfen werden. Eine Abtrennung der Stickstoffheterocyclen von den aliphatischen oder cycloaliphatischen Lösungsmitteln ist problemlos gewährleistet, vor allem wenn man bei der Auswahl dieser Lösungsmittel darauf achtet, dass eine Differenz der Siedepunkte dieser Lösungsmittel und der Stickstoffheterocyclen besteht.

Die zu raffinierenden Gemische, die Wasser und ein oder mehrere Stickstoffheterocyclen enthalten, werden in einem ersten Verfahrensschritt mit einem unpolaren Kohlenwasserstoff oder -gemisch und mit einem wasserlöslichen Salz oder -gemisch versetzt und intensiv miteinander vermischt, wobei sich das unpolare Lösungsmittel in dem wässrigen Gemisch verteilt und sich das Salz entsprechend seiner Löslichkeit im Wasser löst.

Als unpolare Kohlenwasserstoffe werden niedermolekulare aliphatische oder cycloaliphatische Verbindungen sowohl einzeln als auch im Gemisch miteinander eingesetzt. Beispiele für diese Alkane sind Pentan, Hexan, Heptan, Octan, Isooctan, Cyclohexan, Cycloheptan, Methyl- oder Dimethylcyclohexan. Da diese Alkane von den Stickstoffheterocyclen destillativ getrennt werden, soll die Auswahl so erfolgen, dass die entsprechenden Siedepunkte soweit auseinanderliegen, dass eine problemlose destillative Trennung gewährleistet ist. Im allgemeinen ist dies bei Einsatz eines handelsüblichen Hexangemisches mit etwa 60% n-Hexan und einem Siedepunkt von etwa 75°C gegeben.

Die aliphatischen oder cycloaliphatischen Lösungsmittel werden in einer Menge von 50 bis 100%, bezogen auf die eingesetzte Basenmischung, verwandt.

Als Salze können im erfindungsgemässen Verfahren alle gut wasserlöslichen Salze oder Gemische derselben eingesetzt werden. Aus wirtschaftlichen Gründen wählt man zweckmässigerweise ein Salz aus der Gruppe der Chloride, Sulfate oder Carbonate der Alkalimetalle Natrium oder Kalium oder des Ammoniums. Die Menge der eingesetzten Salze richtet sich nach deren Löslichkeit im Wasser. Der angestrebte Phasentrenneffekt ist umso deutlicher, je mehr Salz in der wässrigen Phase, der Sole, gelöst ist. Man wird daher möglichst hochkonzentrierte wässrige Lösungen anstreben. Da andererseits ein Bodensatz aus Salz nicht störend auf das Verfahren wirkt, ist eine Salzdosierung über die Sättigungsgrenze der wässrigen Phase hinaus nicht nachteilig.

Die angestrebte hohe Salzkonzentration legt es nahe, keine Salzlösungen zuzudosieren, sondern feste Salze, die aber durchaus Kristallwasser enthalten können.

Das Durchmischen der Komponenten erfolgt zweckmässigerweise durch Rühren.

Während des Mischvorganges löst sich das Salz im Wasser und die Stickstoffheterocyclen werden von dem aliphatischen oder cycloaliphatischen Lösungsmittel aufgenommen. Wird der Durchmischungsvorgang abgebrochen, so trennt sich die organische Phase von der schweren wässrigen Phase, der Sole, ab.

Es wurden gefunden, dass die Phasentrennung umso schneller erfolgt, je höher die Temperatur ist. Überraschenderweise wird das Verhältnis der Phasen nicht durch die Temperatur beeinflusst. Um einen möglichst raschen Verfahrensablauf zu erzielen, wird daher das Phasengemisch während des Durchmischungsvorganges bis auf eine Temperatur unterhalb des Siedebeginnes einer der Komponenten erwärmt.

Bei Verwendung von Pentan (Kp 36°C) als Lösungsmittel kann daher nur bis 35°C erwärmt werden, während bei Einsatz von relativ hochsiedenden Alkanen wie Dimethylcyclohexan (Kp ~ 120°C) bis zur Siedetemperatur des Wassers erhitzt werden kann.

Nach der Trennung der Phasen in einer Scheidevorrichtung wird die organische Phase, die etwa 98,5 - 99% der Stickstoffheterocyclen enthält, destillativ aufgearbeitet. Hierbei können sowohl das Lösungsmittel von den Stickstoffbasen als auch einzelne Stickstoffbasen voneinander abgetrennt werden. Das zurückgewonnene Lösungsmittel kann im gleichen Prozess wieder verwendet oder aber auch zur Extraktion der Sole eingesetzt werden.

Die Sole enthält noch 1 - 1,5% der Stickstoffbasen. Die Gewinnung dieser relativ geringen Mengen ist weniger aus wirtschaftlichen, denn aus Umweltgründen notwendig.

Es hat sich aber überraschenderweise gezeigt, dass eine Extraktion dieser Sole mit einem aliphatischen oder cycloaliphatischen Lösungsmittel den Gehalt der Sole an Stickstoffbasen unter 0,1% senkt.

Für diese Extraktion können die gleichen Lösungsmittel wie im ersten Verfahrensschritt verwendet werden. Die erhaltenen Extrakte können entweder direkt destillativ aufgearbeitet oder als Lösungsmittel in dem ersten Verfahrensschritt eingesetzt und danach erst destillativ aufgearbeitet werden. In Anbetracht der geringen Basengehalte empfiehlt sich die letztere Variante. Die extrahierte Sole ist praktisch basenfrei und kann als Abwasser verworfen werden.

Es ist ein weiterer Vorteil des erfindungsgemässen Verfahrens, dass es jederzeit kontinuierlich durchgeführt werden kann, wie dies auch mit Hilfe des Beispiels 2 gezeigt wird.

In den Beispielen 1 und 4 sind typische Verfahrensabläufe zur Raffination von Rohbasen beschrieben. Beispiel 5 ist ein Vergleichsbeispiel.

Alle aufgeführten Prozentangaben sind Angaben in Gewichtsprozenten.

### Beispiel 1

Bei der Herstellung von Piperidin gemäss dem US-Patent 3,163,652 fällt ein Rohpiperidin an, das etwa 30% Wasser enthält.

100 Gewichtsteile dieses Rohpiperidins, das aus etwa 47% Piperidin, etwa 20% höhersiedenden Basen und etwa 33% Wasser besteht, werden mit 100 Gewichtsteilen Cyclohexan und 45 Gewichtsteilen Pottasche versetzt und unter intensivem Rühren auf eine Temperatur von 75°C gebracht. Danach wird der Rührvorgang beendet und das Gemisch bei dieser Temperatur belassen.

Innerhalb von 10 min erfolgt eine Phasentrennung, wobei sich die wässrige Phase unten absetzt.

Die Phasen werden getrennt. Die organische Phase wird destillativ aufgearbeitet.

Es werden erhalten:

| | | |
|---|---|---|
| Cyclohexan | 95,4 GT | (Gewichtsteile) |
| Piperidin | 46,5 GT | |
| N-Alkylpiperidine | 18,75 GT | |
| Tetrahydrofuranamine | 0,95 GT | |
| Destillationsrückstand | 7,95 GT | |

Die wässrige Phase wird zur Beseitigung der Spurenanteile mit 50 GT Cyclohexan ausgeschüttet. Die so erhaltene Wasserphase ist nahezu basenfrei (0 - < 0,1% Basengehalt). Die Cyclohexanphase wird destilliert und ergibt 0,85 GT Stickstoffbasen.

### Beispiel 2

In den Mittelteil einer Extraktorkolonne werden kontinuierlich Rohpiperidin gemäss Beispiel 1, Hexangemisch und Natriumchlorid im Gesamtsverhältnis 100 : 100 : 14 eingespeist, innig vermischt und auf eine Temperatur von 70°C gebracht.

Bei diesen Bedingungen scheiden sich am Kopf der Kolonne ein Rohbasen-Hexangemisch und am Boden eine wässrige Phase ab uns werden kontinuierlich abgezogen. Das Verhältnis der organischen Phase zur wässrigen Phase beträgt 160,9 : 53,1. Der Rohbasen-Hexanstrom ist praktisch wasserfrei und enthält 98,6% der Gesamtbasen. Er wird kontinuierlich destillativ aufgearbeitet und liefert die wasserfreien Stickstoffheterocyclen und einen Hexangemischstrom. Dieses Hexan wird einer Extraktionskolonne zugeführt, in der die abgetrennte wässrige Phase, die 1,4% der Gesamtbasen enthält, kontinuierlich extrahiert wird.

Die dabei entstehende Wasserphase enthält dann einen Basenanteil von weniger als 0,1% und wird verworfen. Die Hexanphase, die geringe Anteile an Basen enthält, wird wieder der Rohpiperidinbasentrennung zugeführt.

### Beispiel 3

Eine Lösung aus 70 g Piperidin und 30 g Wasser wird mit 100 g Hexangemisch versetzt. Dazu werden 12 g festes Natriumsulfat (wasserfrei) gegeben. Dieses Gemisch wird unter Rühren auf 68°C erhitzt und danach der Rührvorgang beendet.

Die sich abscheidende, obenstehende, organische Phase wird abgezogen. Sie ist wasserfrei. Durch fraktionierte Destillation werden 69,4 g reines Piperidin erhalten. Die Wasserphase enthält 1,2% Piperidin.

### Beispiel 4

Zu einer Lösung aus 70 g α-Picolin in 30 g Wasser werden 100 g Heptan und 10 g Calciumchlorid (wasserfrei) gegeben, unter Rühren auf 95°C erwärmt und danach der Rührvorgang beendet.

Aus dem sich abscheidenden Picolin-Heptan-Gemisch werden durch Destillation 69,3 g reines α-Picolin erhalten.

Die wässrige Phase wird mit 50 ml Heptan extrahiert und ist danach praktisch picolinfrei.

### Beispiel 5 (Vergleichsbeispiel)

100 g einer Lösung aus 70 g Piperidin und 30 g Wasser werden mit 15 g Natriumchlorid versetzt und unter Rühren auf 75°C erhitzt. Danach wird der Rührvorgang beendet und das Gemisch auf dieser Temperatur belassen. Innerhalb von 30 min tritt eine Phasentrennung ein.

Die sich abscheidende organische Phase enthält 49 g Wasser. Die wässrige Phase enthält 5% Piperidin.

Wird das auf 75°C erhitzte Gemisch nicht bei dieser Temperatur gehalten, so tritt während des Abkühlvorganges eine Phasenverschiebung ein, bei der die wässrige Phase nahezu verschwindet.

### Patentansprüche

1. Verfahren zur Raffination von Stickstoffheterocyclen aus wasserhaltigen Gemischen, dadurch gekennzeichnet, dass die wasserhaltigen Gemische mit einem aliphatischen oder cycloaliphatischen Lösungsmittel oder -gemisch und mit einem wasserlöslichen Salz oder Salzgemisch versetzt und unter intensivem Mischen auf eine Temperatur im Bereich von 35 bis 100°C gebracht werden, wonach die Phasen getrennt werden, die organische Phase destillativ aufgearbeitet und die wässrige Sole mit einem aliphatischen oder cycloaliphatischen Lösungsmittel extrahiert und dieser Extrakt destillativ aufgearbeitet wird.

2. Verfahren nach Anspruch 1, dadurch gekennzeichnet, dass wasserlösliche Salze, Alkalichloride, -sulfate oder -carbonate oder Gemische verwendet werden.

3. Verfahren nach Anspruch 1 und 2, dadurch gekennzeichnet, dass als Lösungsmittel niedrigsiedende Alkane verwendet werden.

4. Verfahren nach Ansprüchen 1 bis 3, dadurch gekennzeichnet, dass als Lösungsmittel Hexangemisch verwendet wird.

5. Verfahren nach Ansprüchen 1, 2 und 4, dadurch gekennzeichnet, dass die Behandlung bei 70°C durchgeführt wird.

6. Verfahren nach Ansprüchen 1 bis 5, dadurch gekennzeichnet, dass als Lösungsmittel für den ersten Verfahrensschritt die organische Phase aus der Soleextraktion verwendet wird.

7. Verfahren nach Ansprüchen 1 bis 5, dadurch gekennzeichnet, dass das Verfahren kontinuierlich durchgeführt wird.

### Claims

1. A process for the refining of heterocyclic compounds with nitrogen in the molecule from mixtures containing water, characterized in that the mixtures containing water are compounded with an aliphatic or cycloaliphatic solvent and a water soluble salt or mixture of salts and heated to a temperature in the range of 35 to 100°C while being intensively mixed after which the phases are separated, the organic phase is worked up by distillation and the aqueous brine is extracted with an aliphatic or cycloaliphatic solvent and this extract is worked up by distillation.

2. A process of claim 1, wherein as water soluble salts alkali metal chlorides, sulfates or carbonates or mixtures are used.

3. A process of claims 1 and 2, wherein as solvents low boiling alkanes are used.

4. A process of claims 1 to 3, wherein as solvent a mixture of hexanes is used.

5. A process of claims 1, 2 and 4, wherein the temperature is 70°C.

6. A process of claims 1 to 5, wherein as solvent for the first step of the process the organic phase of the extraction of the brine is used.

7. A process of claims 1 to 5, wherein the process is run continuously.

### Revendications

1. Procédé de raffinage d'hétérocycles azotés à partir de mélanges aqueux, caractérisé par le fait que les mélanges aqueux sont mélangés avec un solvant ou un mélange de solvants aliphatiques ou cycloaliphatiques et avec un sel ou un mélange de sels solubles dans l'eau, puis amenés, sous agitation intense à une température située dans le domaine de 35 à 100°C, après quoi on sépare les phases, on traite la phase organique par distillation et on extrait la sole aqueuse à l'aide d'un solvant aliphatique ou cycloaliphatique, l'extrait ainsi obtenu étant traité à son tour par distillation.

2. Procédé selon la revendication 1, caractérisé par le fait que l'on utilise, à titre de sels solubles dans l'eau, des chlorures alcalins, des sulfates alcalins ou des carbonates alcalins ou des mélanges.

3. Procédé selon les revendications 1 et 2, caractérisé par le fait qu'à titre de solvants, on utilise des alcanes à bas point d'ébullition.

4. Procédé selon les revendications 1 à 3, caractérisé par le fait qu'à titre de solvant, on utilise un mélange d'hexanes.

5. Procédé selon les revendications 1, 2 et 4, caractérisé par le fait que le traitement est effectué à 70°C.

6. Procédé selon les revendications 1 à 5, caractérisé par le fait qu'à titre de solvant pour la première étape du procédé, on utilise la phase organique provenant de l'extraction de la sole.

7. Procédé selon les revendications 1 à 5, caractérisé par le fait que le procédé est mise en oeuvre en continu.